**Europäisches Patentamt**

**European Patent Office**

**Office européen des brevets**

(19)

(11) Veröffentlichungsnummer: **0 054 222**
**B1**

(12) **EUROPÄISCHE PATENTSCHRIFT**

(45) Veröffentlichungstag der Patentschrift:
02.09.87

(51) Int. Cl.⁴: **G 01 N 33/497**

(21) Anmeldenummer: **81110070.0**

(22) Anmeldetag: **02.12.81**

(54) **Verfahren zum Sicherstellen gültiger Atemproben zum Bestimmen der Blutalkoholkonzentration.**

(30) Priorität: **12.12.80 DE 3046774**

(43) Veröffentlichungstag der Anmeldung:
**23.06.82 Patentblatt 82/25**

(45) Bekanntmachung des Hinweises auf die Patenterteilung:
**02.09.87 Patentblatt 87/36**

(84) Benannte Vertragsstaaten:
**AT BE CH DE FR GB IT LI LU NL SE**

(56) Entgegenhaltungen:
**DE-A-2 610 578**
**DE-A-2 746 078**
**DE-A-2 853 628**
**DE-B-2 816 499**
**US-A-3 792 272**

**Die Akte enthält technische Angaben, die nach
dem Eingang der Anmeldung eingereicht wurden
und die nicht in dieser Patentschrift enthalten sind.**

(73) Patentinhaber: **Siemens Aktiengesellschaft Berlin
und München, Wittelsbacherplatz 2, D-8000
München 2 (DE)**

(72) Erfinder: **Slemeyer, Andreas, Dr., Imberstrasse 33,
D-7500 Karlsruhe 41 (DE)**

EP 0 054 222 B1

LIBER, STOCKHOLM 1987

## Beschreibung

Die Erfindung betrifft ein Verfahren zur Bestimmung der Blutalkoholkonzentration gemäß Oberbegriff des Anspruchs 1.

Grundlage aller Meßverfahren, die die Blutalkoholkonzentration (BAK) über die Analyse der Atemalkoholkonzentration (AAK) bestimmen, ist die Annahme, daß zwischen der Alkoholkonzentration des Blutes und derjenigen der Exspirationsluft ein feststehendes Verteilungsverhältnis besteht. Dies gilt jedoch nur für diejenigen Anteile der Exspirationsluft, die sich im Gleichgewicht mit der Alveolarkonzentration befinden. Für eine korrekte Bestimmung der BAK aus der analyse der AAK ist daher die Abgabe einer Atemprobe, die in der endexspiratorischen Phase aus überwiegend Alveolarluft besteht, als notwendige Voraussetzung anzusehen. Näherungsweise kann dies anhand des zeitlichen Verlaufs der Exspirationskonzentration dadurch festgestellt werden, daß diese gegen Ende der Exspiration eine erkennbare Plateaubildung zeigt.

Hierauf stützen sich auch eine Reihe von Verfahren, die den zeitlichen Verlauf der Konzentrationssteigung erfassen und bei Unterschreitung eines Schwellwertes die abgegebene Atemprobe als gültig bezeichnen (DE-A-2 610 578, DE-B-2 746 078). Dies ist aber noch an die Bedingung geknüpft, daß ein ununterbrochener Mindestvolumenstrom über eine Mindestzeit abgegeben wird, woraus ein erforderliches Mindestvolumen resultiert. Es findet also eine ausschließlich logische Verknüpfung der Meßgrößen Konzentrationsanstieg und Mindestvolumen Anwendung, ohne Berücksichtigung der körperlichen Konstitution des Probanden. Damit auch Personen mit geringer Vitalkapazität (VK) eine gültige Messung erzielen können, darf daher das Mindestvolumen bzw. der Mindestvolumenstrom nicht zu hoch angesetzt werden. Personen mit großer Vitalkapazität ist es somit möglich, durch Reduzierung ihres Volumenstromes auf das geforderte Mindestmaß den zeitlichen Konzentrationsanstieg soweit zu verringern, daß er den Schwellwert unterschreitet, obwohl noch ein erheblicher Teil an Lungenrestvolumen vorhanden wäre. In diesem Fall wurde die zur Analyse benutzte Atemprobe deutlich unter der alveolaren Gleichgewichtskonzentration liegen und eine geringere Alkoholisierung vermuten lassen.

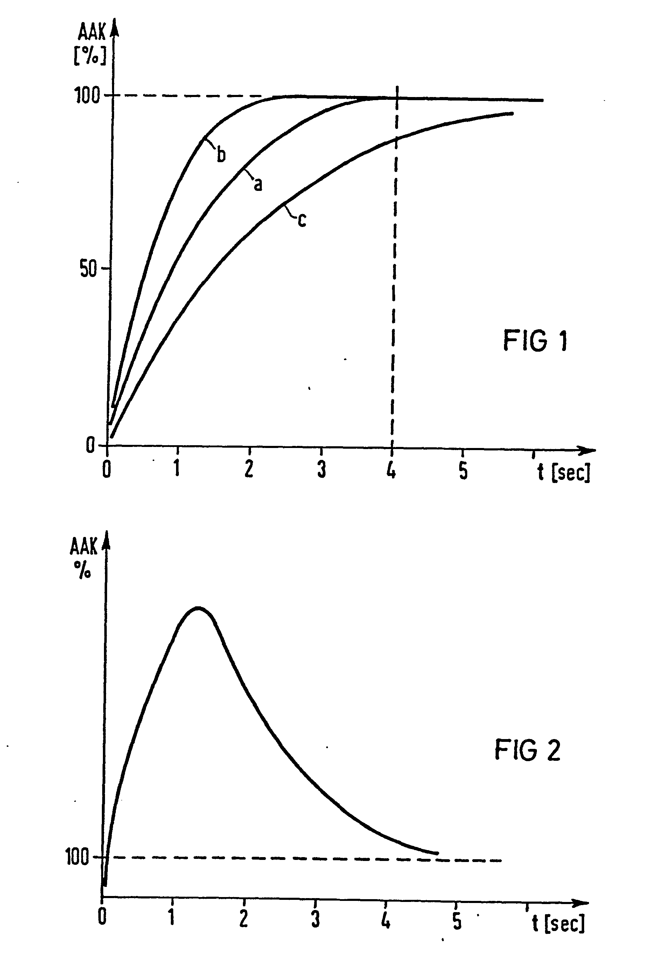

Der Einfluß der Ventilation (Atmung) vor der Probenabgabe auf den Verlauf der ausgeschiedenen Atemalkoholkonzentration ist in Fig. 1 dargestellt. Kurve a beschreibt den Konzentrationsanstieg, wie er sich nach normaler Einatmung vor dem Test ergeben würde. Nach anfänglich großer Steigung flacht die Kurve bereits nach wenigen Sekunden ab und mündet in die asymptotische Grenzgerade ein, die der alveolaren Gleichgewichtskonzentration entspricht. Wird vor der Probenabgabe die Atmung für mehrere Sekunden unterbrochen, so bewirkt dies einen noch steileren Anstieg und somit ein rascheres Erreichen der Gleichgewichtskonzentration; siehe Kurve b.

Ein gegensätzliches Verhalten ergibt sich für den Fall von forcierter Ventilation oder Hyperventilation vor dem Test. Die Kurve c weist im Vergleich zur Normalatmung eine wesentlich größere Steigung in der endexspiratorischen Phase auf, wobei der tatsächliche Wert der AAK auch nach der Überschreitung einer Mindestzeit, hier zum Beispiel 4 sec, nicht mehr erreicht werden kann. Auch bei Hyperventilation kann der zeitliche Konzentrationsanstieg durch Reduzierung des Atemluftdurchsatzes soweit verringert werden, daß der geforderte Schwellwert für eine gültige Atemprobe erreicht wird.

Alle bisher genannten Störeinflüsse, wie unzureichendes Probenvolumen oder Hyperventilation, führen grundsätzlich zu einer Unterbewertung der Atemalkoholkonzentration. Anders verhält es sich bei der Anwesenheit von Mundrestalkohol. Dies kann dann der Fall sein, wenn die Zeit zwischen Trinkende und Test weniger als 15 Minuten beträgt. Meßtechnisch würde sich dabei ein Verlauf entsprechend Fig. 2 ergeben, der durch ein starkes Überschwingen gekennzeichnet ist. Selbst gegen Ende der Exspiration kann der Einfluß von Mundrestalkohol noch so stark sein, daß ein weit über der tatsächlichen Atemalkoholkonzentration liegender Wert vorgetäuscht wird. Das gleiche gilt auch für Luftanteile, die nach Aufstoßen aus dem Magen sich mit der Exspirationsluft vermischen. Hieraus resultierende Fehlbeurteilungen des Alkoholisierungsgrades sollten meßtechnisch erkannt und ausgeschieden werden können.

Der Erfindung vorausgehende Untersuchungen haben gezeigt, daß die wichtigste Einflußgröße auf den Konzentrationssteigungsverlauf während der Exspirationsphase in dem vorangegangenen Inspirationsvolumen liegt. Dabei zeigt sich bei gleichartiger Ausatmung eine quasilineare Abhängigkeit zwischen Inspirationstiefe und Steigung. Dies ist durch die Austauschvorgänge von Äthanol in den zuführenden Atemwegen bedingt. Die Einatmung alkoholfreier Luft führt zunächst zu einer starken Reduzierung der Oberflächenkonzentration, die im wesentlichen nur dadurch wieder rückgängig gemacht werden kann, daß das gleiche Volumen an alkoholgesättigter Alveolarluft bei der Ausatmung wieder vorbeiströmt. Dabei wird der Exspirationsluft solange Alkohol entzogen, bis das Konzentrationsgleichgewicht in den zuführenden Atemwegen wiederhergestellt ist. Eine Verdoppelung des Einatmungsvolumens erfordert somit auch eine Verdoppelung des Ausatmungsvolumens, um gegen Ende der Exspiration eine Plateaubildung im Konzentrationsverlauf einzuleiten.

Aus den Untersuchungen ist weiterhin zu erkennen, daß unter der Bedingung von gleichem Inspirations- und Exspirationsvolumen im Mittel nur 80 % des alveolaren Gleichgewichtskonzentrationswertes erreicht werden, weshalb vom Probanden noch weiteres Exspirationsvolumen gefordert werden muß. Dazu ist dieser jedoch nur dann in der Lage, wenn er nicht mehr als ungefähr 40 % seiner Vitalkapazität eingeatmet hat. Das zusätzlich notwendige Volumen vergrößert sich außerdem noch mit steigendem Volumen der Meßkammer des Analysegerätes, weshalb diese so klein wie möglich ausgebildet sein muß

Hieraus wird erkennbar, daß die Erfüllung der Mindestvolumenbedingung nur dann bei Personen mit vergleichbarer Vitalkapazität Anwendung finden kann, wenn außerdem die gleiche Einatmungstiefe vorliegt. Ähnliches gilt für die Überwachung des zeitlichen Konzentrationsanstiegs. Aus ihm lassen sich nur dann Aussagen über die Annäherung an die Gleichgewichtskonzentration ableiten, wenn der dabei abgegebene Volumenstrom in einer bestimmten Relation zur Vitalkapazität des Probanden steht. Um eine gültige Probenabgabe feststellen zu können, müßten daher sowohl die Vitalkapazität als auch das In- und Exspirationsvolumen gemessen werden. In der Praxis ist dies mit den Mitteln des Standes der Technik kaum möglich. Das Wissen um die oben genannten Zusammenhänge konnte deshalb bisher nicht dazu verwendet werden, ungültige Probenabgaben aus der Beurteilung des tatsächlichen Alkoholisierungsgrades auszuscheiden.

Demgegenüber ist es Aufgabe der Erfindung, mit in der Praxis durchführbaren meßtechnischen Methoden solche zu unrichtigen Ergebnissen führenden Atemproben aus der Beurteilung auszuscheiden. Aus der Analyse von ausschließlich Exspirationsluft soll mit Hilfe einer einfachen Rechenvorschrift eine Aussage über den Grad der Annäherung an die Gleichgewichtskonzentration abgeleitet werden. Die Lösung für diese Aufgabe ist durch die Merkmale des Anspruchs 1 gekennzeichnet.

Die dort angegebene Beziehung

$$S(t) = \frac{d\,AAK(t)}{d\,V(t)} \cdot \frac{V(t)}{AAK(t)}$$

setzt die prozentuale Konzentrationsänderung in das Verhältnis zur prozentualen Volumenänderung, wobei der zweite Term zur Normierung dient, um von der Höhe der Atemalkoholkonzentration AAK und der Größe des Exspirationsvolumens V unabhängig zu werden.

Ein besonderer Vorzug liegt noch darin, daß diese Gleichung unabhängig von der tatsächlichen Vitalkapazität des Probanden gilt.

Zur Erläuterung der Erfindung dienen die Zeichnungen. In diesen ist

Fig. 1 die bereits erläuterte Darstellung der Abhängigkeit des zeitlichen Konzentrationsverlaufs in der Atemluft von der Ventilation vor dem Test,

Fig. 2 die bereits erläuterte Darstellung des Konzentrationsverlaufs bei vorhandenem Mundrestalkohol,

Fig. 3 die Darstellung des Verlaufs der auf das Volumen bezogenen Steigung S(t) als Funktion des Exspirationsvolumens, wobei das Inspirationsvolumen den Parameter bildet,

Fig. 4 eine Darstellung entsprechend Fig. 3, jedoch aufgetragen über der ausgeschiedenen AAK, und

Fig. 5 die Darstellung von Beispielen für die Anwendung des Steigungskriteriums mittels verschiedener Schwellwerte.

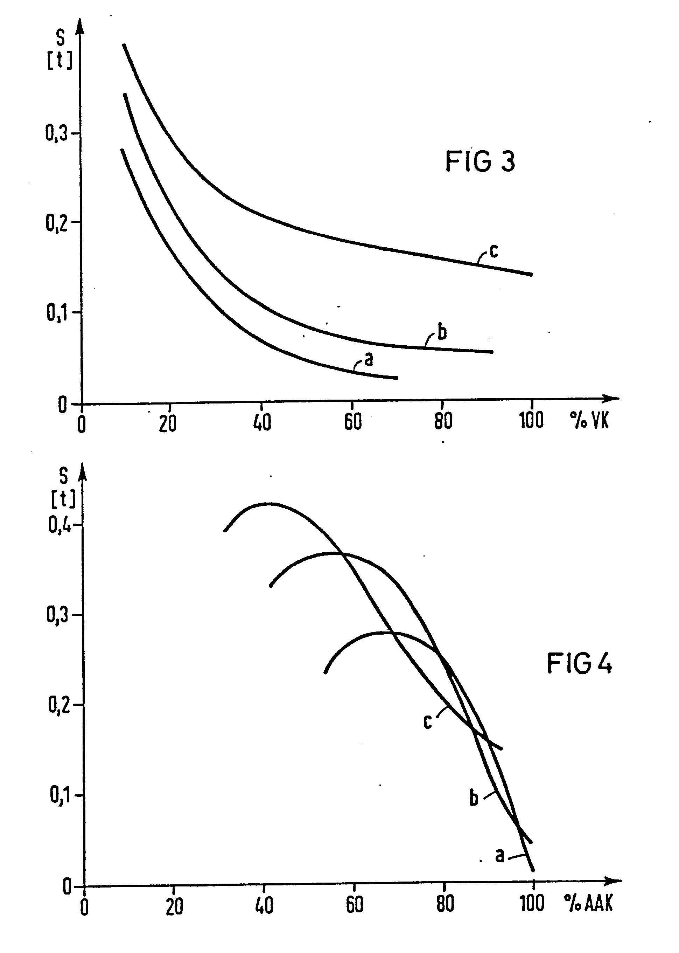

Mit der vorstehend genannten erfindungsgemäßen Verwendung der Steigung der Exspirationskonzentration als einzigen Entscheidungsparameter wird der folgenden Beobachtung entsprochen: Trägt man den Verlauf der Atemalkoholkonzentration über der relativen Vitalkapazität auf, so zeigt sich bei gegebenem relativen Inspirationsvolumen, daß das Anstiegsverhalten sowohl von der Atemströmungsgeschwindigkeit als auch von der tatsächlichen Vitalkapazität der Versuchsperson unabhängig ist. Dieser Zusammenhang ist in Fig. 3 dargestellt. Parameter ist das auf die Vitalkapazität bezogene relative Inspirationsvolumen. Kurve a gilt für 20 % VK, die Kurven b und c für 40 bzw. 100 % VK. In den beiden erstgenannten Fällen enden die Kurven bei 70 bzw. 90 % der VK, was der Summe aus Inspirationsvolumen und Reservevolumen entspricht. Der Kurve c ging eine vollständige Aus- und Einatmung voraus (forcierte Ventilation). Mit zunehmendem Inspirationsvolumen erhöht sich der Wert der Steigung S in der endexspiratorischen Phase, da die Austauschprozesse in den Atemwegen noch nicht vollständig abgeschlossen sind.

Aus dem Steigungsverlauf in Abhängigkeit von der ausgeschiedenen AAK läßt sich ein eindeutiger Zusammenhang zwischen der Größe von S und der Annäherung an die alveolare Gleichgewichtskonzentration ableiten. So werden zum Beispiel 90 % der tatsächlichen AAK nur dann erreicht bzw. überschritten, wenn die Bedingung $S \leqslant 0{,}15$ erfüllt ist (Kurven a und b in Fig. 4). Bei unvollständiger Probenabgabe oder forcierter Ventilation ist dies jedoch nicht möglich.

Ein besonderer Vorzug des erfindungsgemäßen Verfahrens besteht darin, daß der Grad der Annäherung an die alveolare Gleichgewichtskonzentration unabhängig von Inspirationsvolumen und Vitalkapazität aus der Größe der Steigung S bestimmt werden kann. Der Vergleich der Größe S mit einem Schwellwert liefert dabei eine Aussage, ob noch weiteres Volumen exspiriert werden muß damit eine korrekte Bestimmung der Atemalkoholkonzentration durchgeführt werden kann.

Eine weitere Anwendung dieses Steigungskriteriums ergibt sich im Falle von Mundrestalkohol. Hier treten zu Beginn der Exspiration kurzzeitig sehr viel höhere Konzentrationswerte auf als im weiren Verlauf. Dadurch errechnen sich für die Größe S negative Werte, aus denen eine solche Situation meßtechnisch sicher erkannt werden kann.

Zur schaltungstechnischen Darstellung der oben genannten Rechenvorschrift sind für die Messung der Zeitverläufe von Atemalkoholkonzentration und Exspirationsvolumen Zeitkonstanten $T_{63}$ von < 0,2 Sekunden erforderlich. Daher wird für die Alkoholmessung ein IR-Analysegerät mit kleinem Meßkammervolumen verwendet, wie es zum Beispiel in DE-B-2 805 972, DE-C-2 659 898 (Prof. Adrian) beschrieben ist. Die Erfassung des Atemvolumens kann zum Beispiel mit Hilfe eines Thermistor-Anemometers vorgenommen werden, wobei Konstanttemperaturbetrieb in Hinblick auf das geforderte Auflösungsvermögen zu bevorzugen ist. Hieraus ergibt sich eine dem Atemvolumenstrom $\dot{V}$ proportionale Meßgröße, aus der durch zeitliche Integration das Atemvolumen V gewonnen werden kann.

Die Berechnung von S kann sowohl unter Verwendung von Analogrechenbausteinen als auch durch digitale Rechenwerke durchgeführt werden Entsprechend dem Stand der Technik ist die digitale Lösung vorzuziehen. Dazu werden die Eingangsgrößen AAK und V zeitlich abgetastet. Ihre Ableitungen lassen sich dann als Differenz zweier aufeinanderfolgender Abtastwerte darstellen. Zur Erhöhung der Rechengenauigkeit in der endexspiratorischen Phase kann eine Mittelwertbildung vorgenommen werden, indem mehrere aufeinanderfolgende Abtastwerte zusammengefaßt werden.

Der Meßvorgang wird dadurch ausgelöst, daß die Betragsdifferenz zweier aufeinanderfolgender Abtastwerte von V größer als ein vorgegebener Schwellwert wird; er wird beendet, sobald dieser Schwellwert zum ersten Mal wieder unterschritten wird. Durch Klappenventile wird dabei eine Einatmung des Probanden aus dem Gerät verhindert. Die Zeitdauer des gesamten Meßvorgangs wird intern erfaßt.

Die Berechnung der volumenbezogenen Steigung erfolgt nun zu jedem Abtastzeitschritt, wobei das Ergebnis mit folgenden Schwellwerten verglichen wird:

a) Positiver Schwellwert S+: Ein Erreichen oder Unterschreiten dieses Wertes signalisiert, daß die abgegebene Atemprobe sich der alveolaren Gleichgewichtskonzentration ausreichend angenähert hat, sie somit als gültig anerkannt werden kann. Die zu diesem Zeitpunkt gemessene Alkoholkonzentration wird nun unmittelbar der Anzeige zugeführt, wodurch der Meßvorgang beendet wird. Es besteht wahlweise auch die Möglichkeit, den Zeitpunkt abzuwarten, an dem die Betragsdifferenz zweier Abtastwerte von V wieder unter den Schwellwert gesunken ist, um das eventuell noch vorhandene Restvolumen für die Probe zu gewinnen.

b) Negativer Schwellwert S-: Wird dieser betragsmäßig überschritten, so erfolgt ein Abbruch des Meßvorganges, verbunden mit einem Hinweis auf vorhandenen Mundrestalkohol.

Der positive Schwellwert S+ läßt sich größenmäßig nicht exakt festlegen. Sein genauer Wert hängt davon ab, welche Genauigkeit die verwendete Apparatur zuläßt und welche Genauigkeit von den gesetzlichen Vorschriften verlangt wird. S+ liegt dann vor, wenn sich die tatsächliche AAK deren Endwert so weit angenähert hat, wie es die Meßgenauigkeit zuläßt und die gesetzlichen Vorschriften verlangen. Die Praxis zeigt, daß S+ dann vorliegt, wenn sich die tatsächliche AAK deren Endwert bis auf etwa 5 Prozent angenähert hat. In absoluten Zahlen bedeutet dies, daß S+ zwischen +0,2 und +0,1, vorzugsweise zwischen +0,15 und +0,1 liegen sollte.

Der negative Schwellwert S- sollte zwischen -0,2 und -0,3 liegen und vorzugsweise -0,3 betragen. Er ist so zu wählen, daß er bei geringem Mundrestalkohol sicher unterschritten wird.

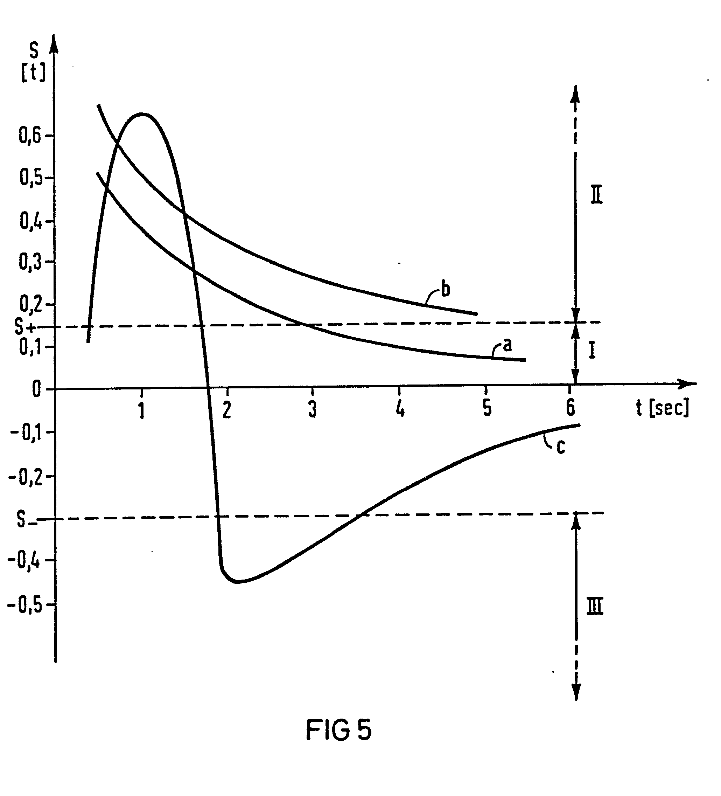

Die Anwendung des beschriebenen Verfahrens soll an drei Beispielen in Fig. 5 erläutert werden. Dazu ist die Ordinate in folgende Bereiche unterteilt:

Bereich I:      $0 < S(t) \leqslant S+$
Bereich II:      $S(t) > S+$
Bereich III:      $S(t) < S-$

Die Übergänge zwischen den Bereichen sind dabei durch Schwellwerte S+ und S- gekennzeichnet.

Bei normaler Ventilation vor dem Test verringert sich im Verlauf der Exspiration die Größe S(t) stetig, bis sie schließlich von Bereich II nach Bereich I wechselt und dort bis zum Ende der Ausatmung verbleibt (Kurve a). Sobald der Schwellwert S+ unterschritten wird, ist eine solche Atemprobe als gültig anzusehen. Bei einem angenommenen S+ = 0,15 wäre dies bereits nach drei Sekunden der Fall. Möglich ist es aber auch, das Ende der Exspiration abzuwarten, das durch ein Unterschreiten eines Mindestatemluftdurchsatzes gekennzeichnet ist.

Kurve b deutet entweder auf Störungen der Austauschvorgänge vor dem Test oder auf unzureichendes Probenvolumen hin. Letzteres wäre aus dem Meßwert des Exspirationsvolumens zu entnehmen. Da S(t) während des gesamten Verlaufs im Bereich II verbleibt, ist diese Messung ungültig und muß wiederholt werden.

Ein Beispiel für den Einfluß von Mundrestalkohol auf den Verlauf der volumenbezogenen Steigung zeigt Kurve c. Charakteristisch hierfür ist das Überstreichen der Bereiche in der Reihenfolge II, I und III. Sobald der Schwellwert S- unterschritten wird (zum Beispiel bei S- = -0,3), kann die Messung abgebrochen werden, da unter diesen Umständen keine physiologisch relevanten Ergebnisse zu erzielen sind. Zusätzlich kann außerdem die zeitliche Ableitung von S(t) gebildet werden. Wenn hierbei zwei Nullstellen in Verbindung mit Vorzeichenwechsel auftreten, so kann hieraus ebenfalls auf Störeinflüsse durch Mundrestalkohol geschlossen werden.

Im Anschluß an den Meßvorgang werden die Meßwerte für Exspirationsvolumen und Zeitdauer angezeigt. Sie stellen im Falle einer positiven Atemprobe eine wichtige Information für die forensische Bewertung des Atemalkoholkonzentrationswertes dar. Falls keine Anzeige des AAK-Wertes erfolgt, so lassen sich aus ihnen Hinweise für das Exspirationsverhalten des Probanden zum Zwecke einer Wiederholungsmessung ableiten.

**Patentansprüche**

1. Verfahren zur Bestimmung der Blutalkoholkonzentration durch Analyse der Atemalkoholkonzentration (AAK) im Exspirationsvolumen, wobei aus dem gemessenen zeitlichen Verlauf der Atemalkoholkonzentration gewonnene Werte mit einem Schwellwert verglichen werden, <u>dadurch gekennzeichnet</u>, daß die Steigung der Exspirationskonzentration S(t) aus der gemessenen Atemalkoholkonzentration (AAK) und dem gemessenen Exspirationsvolumen (V) pro Zeiteinheit nach der Formel

$$S(t) = \frac{d\,AAK(t)}{d\,V(t)} \cdot \frac{d\,V(t)}{AAK(t)}$$

errechnet wird und die Meßwerte nur dann zur weiteren Auswertung herangezogen werden, wenn S(t) zwischen einem vorgegebenen positiven Schwellwert S+ und einem vorgegebenen negativen Schwellwert S- liegt.

2. Verfahren nach Anspruch 1, <u>dadurch gekennzeichnet</u>, daß als positiver Schwellwert S+ ein Wert zwischen +0,2 und +0,1 vorgegeben wird.

3. Verfahren nach Anspruch 1, <u>dadurch gekennzeichnet</u>, daß als negativer Schwellwert S- der Wert -0,3 vorgegeben wird.

**Claims**

1. A method of determining blood alcohol concentration by analysis of the breath alcohol concentration (AAK) in the volume of breath expired, wherein the values obtained by measuring the variation per unit time of the alcohol concentration in the breath are compared with a threshold value, <u>characterised in</u> that the increase in the expired concentration S(t) is calculated from the measured alcohol concentration in the breath (AAK) and the measured volume (V) expired per unit time according to the formula

$$S(t) = \frac{d\,AAK(t)}{d\,V(t)} \cdot \frac{V(t)}{AAK(t)}$$

and the measured values are only then called upon for further evaluation when S(t) lies between a given positive threshold value S+ and a given negative threshold value S-.

2. A method as claimed in Claim 1, <u>characterised in</u> that a value between +0.2 and +0.1 is set in advance as positive threshold value.

3. A method as claimed in Claim 1, <u>characterised in</u> that the value -0.3 is set in advance as negative threshold value.

**Claims**

1. Procédé de détermination du taux d'alcoolémie par analyse du taux d'alcool de l'haleine (AAX) dans le volume expiré, en comparant à une valeur de seuil les valeurs obtenues à partir de la courbe du taux d'alcool de l'haleine en fonction du temps, caractérisé en ce qu'il consiste à calculer la pente du taux à l'expiration S(t) à partir du taux d'alcool de l'haleine (AAK) qui est mesuré et du volume d'expiration (V) mesuré par unité de temps suivant la formule

$$S(t) = \frac{d\,AAK(t)}{d\,V(t)} \cdot \frac{V(t)}{AAK(t)}$$

et à n'exploiter ensuite les valeurs de mesure que si S(t) est compris entre un seuil positif S+ prescrit et un seuil négatif S- prescrit.

2. Procédé suivant la revendication 1, caractérisé en ce qu'il consiste à donner au seuil positif S+ une valeur comprise entre +0,2 et +0,1.

3. Procédé suivant la revendication 1, caractérisé en ce qu'il consiste à donner au seuil négatif S- la valeur de -0,3.

FIG 1

FIG 2

FIG 3

FIG 4

0 054 222

FIG 5

5